# EUROPEAN PATENT SPECIFICATION

(11) **EP 4 486 140 B1**
(45) Date of publication and mention of the grant of the patent: **02.12.2026**
(21) Application number: 23713143.8
(22) Date of filing: 28.02.2023
(51) Int. Cl.: A61K 35/00, A61P 3/00, A23L 7/104, A23L 31/15, A61K 9/16, A61P 9/00

(54) **NUTRACEUTICAL OR PHARMACEUTICAL COMPOSITION COMPRISING AN EXTRACT OF RED YEAST RICE**
NUTRAZEUTISCHE ODER PHARMAZEUTISCHE ZUSAMMENSETZUNG MIT EINEM EXTRAKT AUS ROTEM HEFEREIS
COMPOSITION NUTRACEUTIQUE OU PHARMACEUTIQUE CONTENANT UN EXTRAIT DE LEVURE DE RIZ ROUGE

(30) Priority: 01.03.2022 IT 202200003224
(43) Date of publication of application: 08.01.2025
(73) Proprietor: Neilos S.r.l., 80063 Piano di Sorrento (NA) (IT)
(72) Inventor: DI MAIO, Umberto, 80063 Piano di Sorrento NA (IT)
(74) Representative: Di Giovine, Paolo
(86) International application number: PCT/IB2023/051853
(87) International publication number: WO 2023/166413

(56) References cited:
- WO-A1-2020/208404
- WO-A1-2021/099976
- WO-A2-2021/090214
- CZ-U1- 31 575
- JP-A- 2021 098 670
- US-A1- 2009 148 433

## Description

The present invention relates to a nutraceutical or pharmaceutical composition effective in the prevention and/or treatment of hypercholesterolemia in both humans and animals.

### Background of the invention

Hypercholesterolemia is a pathological condition characterized by the presence of high levels of lipids (such as cholesterol, triglycerides or phospholipids) in the blood circulation. It is considered the main factor of atherosclerotic and cardiovascular risk since the excessive increase in the levels of LDL cholesterol has been associated to a higher coronary risk. Therefore, the decrease in the LDL levels seems to be the best approach to be adopted for preventing the cardiovascular disorders. The forms of familiar hypercholesterolemia (FH) are characterized by a hyperproduction of cholesterol at liver level, genetically determined and due, in most cases, to a mutation of the gene which codifies for the cell receptor of LDLs which, consequently, is no longer able to bind the circulating LDL particles nor to remove them from the blood circulation. In the developed countries, dyslipidaemias very often are a consequence of the so-called metabolic syndrome and then attributable to unhealthy lifestyle and/or eating habits. This last type of dyslipidaemia is characterized, apart from high levels of LDL and low levels of HDL, even by obesity, insulin resistance, hypertension and diabetes of type 2. Moreover, epidemiological studies have highlighted the involvement of other factors such as the plasmatic increase in fibrinogen and in the coagulation factors, the increase in the platelet activation and modifications of the LDL oxidative state. The pharmacological treatment of election for controlling dyslipidaemias consists in the administration of statins alone or in association with other active principles as inhibitors of the cholesterol absorption or inhibitors of PCSK9, an enzyme adjusting the degradation of LDL receptors. However, the therapy with statins has considerable limits due to the severe side effects at muscle and liver level (for example, myopathies, rhabdomyolyses, alteration in the liver functionality and increase in transaminases) and to pharmaco-resistance and intolerance phenomena. In case of patients resistant to statins, an inadequate (or missing) decrease in the levels of LDL cholesterol takes place, accompanied by the appearance of the side effects generally due to polymorphism of HMGCo-A reductase, of glycoprotein P, of cholesterol 7 α-hydroxylase, to apolipoprotein E, of lipoprotein A or of LDL receptor, whereas in the intolerant patients the use of statins is absolutely not recommended, since the side effects appear quickly and in their most severe form. Another factor to be considered is that the treatment with statins is suitable for patients having high or very high cardiovascular risk and not for subjects having light or moderate cardiovascular risk and have LDL levels not far from the norm thereto a simple lifestyle change is generally recommended. Therefore, there is still the need for solutions for the prevention and/or treatment of cholesterolemia and, more generally, for controlling the cardiovascular risk in subjects resistant or intolerant to statins or who have a low or moderate cardiovascular risk. CZ31575 U1 discloses a carrier for enhancing the bioavailability of Monacolin K and other active substances of Monascus purpureus biomass extract. The Monascus purpureus biomass extract components are in the range of 10 nm to 100 µm, made up of components of the lecithin ethanol extract in an amount of from 1 to 95% by weight and chitosan in an amount of from 1 to 95% by weight.

### GLOSSARY

The terms used in the present description are as generally understood by the person skilled in the art, unless otherwise indicated.

Under the term "extract", in the context of the present description, any product is meant attributable to a plant drug including all products deriving from mechanical treatments (pulverization, grinding, mixing and/or other methods) or from extractive treatments (extraction with solvent, distillation and/or other specific methods) performed on a drug.

### DETAILED DESCRIPTION OF THE INVENTION

### RED YEAST RICE

The red yeast rice (RYR) is a nutraceutical product obtained from fermentation of white rice with Monascus purpureus yeast responsible for the typical colour and for the production of important secondary metabolites. The so-enriched rice includes a series of bioactive compounds thereamong sterols, isoflavones, unsaturated fatty acids and monacolins. Among monacolins, monacolin k is the most abundant and interesting. It is known even as lovastatin since the two molecules have an almost analogous structure. In the red yeast rice, monacolin k is present both in lactone form and in hydrolyzed form. Generally, at low ph values the lactone form prevails which converts spontaneously into the active form of hydroxy acid with neutral or basic pH. In the monacolin metabolism the enzymes of the family of cytochrome 450 and glycoprotein P are involved, therefore such molecule could cause interactions with the substances or the active principles usually metabolized by these routes. Monacolin k represents the active principle responsible for the healthy effect which the red yeast rice has on hypercholesterolemia. Thanks to its structural features the above-mentioned molecule, as well as the statins, is capable of inhibiting the hydroxymethylglutaryl-CoA reductase enzyme (HMGCo-A reductase) which converts hydroxymethylglutaryl-CoA into Mevalonate and which represents the limiting enzyme in the biosynthesis process of cholesterol. The main side effects caused by statins are due to the fact that the pharmacological treatment with the same causes the depletion of important metabolites such as Mevalonate, the mitochondrial coenzyme Q and the regulatory proteins binding GTP, with consequences on the cellular energy metabolism, the mitochondrial function and the intracellular protein signalling pathways. By taking into consideration this factor, the treatment with red rice results to be particularly advantageous since monacolin, even if it exerts the same action of the statins on the HMGCo-A reductase enzyme, has a markedly lower effect as far as the depletion of the cellular metabolites is concerned. Considering the structural overlapping existing between monacolin k and lovastatin, nowadays, the use of RYR in adjusting the plasmatic levels of cholesterol and in the prevention of the cardiovascular disorders is still very controversial, above all as far as the statin-sensitive patients are concerned since there is a certain fear of incurring the same side effects. Despite everything, the clinical data collected over the last years show that, among the subjects using RYR-based supplements, the appearance of serious side effects is lower than 0.1%. Moreover, there is a great number of clinical trials demonstrating the positive effect of monacolin in the decrease in LDL plasmatic levels and in the appearance of cardiovascular events. In 2011, EFSA officially declared that there is a cause-effect relationship between the consumption of 10 mg/die of monacolin and the decrease in the levels of LDL cholesterol in the blood. This value was determined by considering two randomized and double-blind studies, controlled with placebo in which one observes a decrease in the total cholesterol and LDL cholesterol levels of 16% and 22%, respectively, by administering 7.5 mg/die of active principle for 12 weeks and a decrease in the above-mentioned outcomes of 20% and 26% respectively by administering a dose containing 11.4 mg/die of monacolin for 8 weeks.

An in-vivo study on experiment animals examined the effect of the extract of red yeast rice on the lipidic profile of the treated mice and on the vascular endothelial inflammation which is one of the phenomena leading to the initiation of the atherosclerotic process. C57BL/6JApoe-/- mice were divided into three groups:
1. Mice with normal diet, without treatment;
2. Mice with high fat diet, without treatment;
3. Mice with high fat diet and treatment with the extract of RYR (0.34 g/kg per day).

The mice of group 2, not treated and subjected to a high fat diet, developed atherosclerotic plaques and high levels of total cholesterol and LDL cholesterol with respect to the group fed with a normal diet. The treatment with the extract reduced the sizes of the plaques by about 15% and the lesion area. Moreover, the results obtained in the treated group highlighted a decrease in the plasmatic levels of cholesterol with respect to the control group and the not treated group. An analysis of the protein expression levels on the vascular, liver and intestinal tissue of the sacrificed mice highlighted that:
- The treatment with the extract of red rice reduced the expression levels of HMGCoA reductase at liver level;
- RYR reduced the expression levels, at aorta level, of TRL2, TRL4 and MAPK, proinflammatory proteins responsible for the activation of the cascades of signalling the inflammation and the activation of some atherosclerotic genes;
- The extract increased the expression levels of JAM-1 and of occludins at intestinal level by demonstrating to improve the intestinal functions of Apoe-/- mice and, then, to decrease the inflammation risk.

The effectiveness of the extract of red yeast rice from Monascus purpureus was tested in a great, double-blind, multicentric, randomized study, controlled with placebo performed on 4870 Chinese patients between 18 and 70 years old with a preceding documented myocardial infarction, with high levels of creatine kinase and with an average level of LDL equal to 129 mg/dL.

The patients were divided into two groups:
- Treated: 600 mg per day of xuezhikang, the extract of Chinese red yeast rice (each tablet contains 300 mg of extract corresponding to 2.5-3.2 mg of monacolin);
- Placebo

The subjects were kept under control for a period of about 4 years and a half and the following was evaluated: the appearance of coronary events, the mortality due to cardiovascular problems and the levels of plasmatic lipids. The results extrapolated from this study showed that in the subjects of the treated group there was a decrease in the levels of LDL cholesterol of about 20% with respect to the placebo group. The total lipidic profile of the patients results to have improved, with a decrease in the levels of triglycerides and an increase in HDL cholesterol. A decrease in the incidence of cardiovascular events and, more generally, of mortality attributable to cardiovascular problems was noted. The study did not find any case of serious side effects.

A metanalysis grouped 20 randomized trials with 6653 participants. The considered studies compared the extract of red rice containing monacolin k (with doses comprised between 4.8 and 24 mg per day) with placebo or with statins. The results showed that the supplementation with RYR decreases the LDL levels with respect to placebo. When the treatment was compared to statins, no differences were found among the groups, thus demonstrating that monacolin K has an effectiveness comparable to the traditional treatment.

Despite the wide use of monacolin K obtained from red yeast rice, the scientific research focuses on new pharmaceutical forms capable of improving the pharmacokinetic profile of monacolin K, thus maximizing the therapeutic effect.

In fact, after oral administration the total bioavailability is about 5%, but it can be increased up to 30-50% if administered during the meal.

It was observed that the cholesterol synthesis and in particular the activity of the HMGCo-A reductase enzyme follows the circadian cycle, with a peak of activity in the night hours. To demonstrate this, there is the fact that the administration of lovastatin (structural analogue of monacolin K) in the evening together with meals results to be more effective than an administration in the morning.

For this reason, a formulation with prolonged release based on an extract of red yeast rice containing monacolin K could be still more effective than a "classical" formulation, since a prolonged release during the night hours would allow to keep under control the enzyme activity still more accurately.

For this reason, the present invention relates to a technologically new formulation based on an extract of red yeast rice containing monacolin K in association with phosphatidylserine, phosphatidylcholine and starch, in particular acetylated modified starch, capable of slowing down the release of monacolin K by improving the absorption and bioavailability thereof with consequent optimization of its therapeutic effect.

Such formulation, the present invention relates to, guarantees controlled release of monacolin K thanks to the combination with the three functional excipients, such as phosphatidylcholine, phosphatidylserine and starch.

In particular, the starch, preferably acetylated modified starch, guarantees the features of controlled release and thanks to the presence of phosphatidylcholine and phosphatidylserine, monacolin k is absorbed in an optimum way.

The present invention then aims at making available a nutraceutical or pharmaceutical composition adapted to carry monacolin K from red yeast rice to improve the effectiveness, safety and compliance in patients with hypercholesterolemia.

The invention also relates to a pharmaceutical product or a food supplement comprising the nutraceutical or pharmaceutical composition according to the invention. In addition to the active principle, red yeast rice containing monacolin k and to the functional excipients starch, phosphatidylcholine and phosphatidylserine, the pharmaceutical product or food supplement of the invention can optionally include additional active principles and functional excipients, which are easily selected by the person skilled in the art based upon the case needs. Even the selection of the carriers, excipients and/or diluents necessary for the formulation of the pharmaceutical product or food supplement in a suitable dosage form is within the usual capabilities of the person skilled in the art.

The nutraceutical or pharmaceutical composition according to the invention is as defined in the enclosed claim 1.

Additional features and advantages of the invention are defined in the depending claims. The claims form integrating part of the present description.

A detailed description of some preferred embodiments of the invention is provided hereinafter.

As shown, the nutraceutical or pharmaceutical composition of the present invention is based on an extract of red yeast rice containing monacolin K as active principle and a combination of functional excipients which provide to the composition a controlled release and increase in the bioavailability of monacolin K. Such combination of functional excipients includes phosphatidylcholine, phosphatidylserine and starch.

The nutraceutical or pharmaceutical composition of the present invention then is particularly effective for carrying and adding red yeast rice containing monacolin K for the prevention and/or treatment of hypercholesterolemia.

The starch is an organic compound of polysaccharide nature consisting of the repetition of glucose units bound by means of α-glycoside bonds. It consists of two types of polymers: amylose, which generally is about 20%, and amylopectin, which generally is about 80%. Amylose is the central portion of the starch granules, it is soluble in very hot water and it consists of glucose molecules bound by means of α-1,4 glycosidic bonds. Amylopectin is a polymer with a high branching degree which forms the external portion of the granules. The monomeric units composing it are joined, in the branching points, by α-1,6 glycosidic bonds. In nature it forms in the green portions of the plants, to be then accumulated in the storage elements, such as tubers, seeds and roots. Thanks to its properties and features, it has always been used industrially.

The starch is particularly important in the food industry, which uses it as thickening agent and in the production of sweeteners such as maltitol and sorbitol. Thanks to its gluing properties it is also used in the production of paper and glues, under the form of starch weld. In the pharmaceutical industry the starch has always been used as excipient and for the formation of coatings, thanks to its binding properties.

Although the starch is used also in its natural form, the firms' interest is mainly directed to the modified starches, that is starch molecules properly modified to meet the needs of the various productive processes in which the starch is used. Such modified starches can be obtained by using as source plants which have been subjected to natural or induced genetic mutations and which then produce starches with altered features. Another strategy is that of modifying the starch, generally deriving from maize, tapioca and rice, by means of chemical treatments (addition of functional groups, treatment with acids and bases), physical treatments (gelatinization) or enzymatic treatments (partial hydrolysis). Dextrins are an example of modified starches obtained by hydrolysis and repolymerization. Such reactions can be implemented by simple thermal degradation or by acid catalysis. The result is to obtain molecules characterized by shorter chains and then partially or totally soluble in water. Examples of dextrins are cyclodextrins and maltodextrins, excipients widely used in nutraceutical and pharmaceutical field.

The nutraceutical and pharmaceutical industry showed great interest against high amylose modified starches (HAS). A starch, in order to be defined as such, must have a percentage of amylose equal at least to 50%. The starch with high content of amylose can be obtained from genetically modified plants or by enriching with amylose starches having low percentages thereof. The strategies used by several firms provide the use of HAS for the preparation of solid pharmaceutical forms or in the coating processes. The HAS have several advantages with respect to other types of starch, for example a better consistency, a higher thermal stability and a greater resistance to humidity and adhesion phenomena. Several strategies were implemented to exploit HAS advantages in the most suitable way. For example, the use of soft gel capsules, in which a certain percentage of gelatine is replaced by the above-mentioned starch, is attributed to Scherer Corporation. The so-obtained capsules result to have a better aspect and greater resistance. Dow Chemical Company can boast of the use of more uniform capsules, with a greater stability in water and to high temperatures thanks to the use of hydroxyalkyl HAS. Upjohn Company boasts of the use of amylose acetate phthalate as coating agents in the gastro-resistant preparations.

In the present invention different types of starch can be used. By way of example one can mention: a chemically not modified pregelatinized maize starch, or an acetylated (modified) pregelatinized maize starch with a high content of amylose which, in the specific case, can reach up to 90% by weight. The percentage of acetylic groups is comprised between 0.5% and 2.5%.

The acetylation is performed for example with acetic anhydride which guarantees to reach a percentage of acetylic groups higher than 0.5% but not higher than 2.5%. The starch pregelatinization treatment consists in dispersing the acetylated starch in water and subjecting the so-obtained dispersion to temperatures ranging from 100 to 130 degrees and to high pressures. The starch granules subjected to such procedure explode and form a gel with a humidity content ranging from 5 to 10%. Once solidified and removed, the so-obtained modified starch can be used in the processes for coating hard and soft capsules and microgranules and it guarantees to obtain a coating which results to be resistant and adequately viscous at the same time, which is capable of masking unpleasant odours and tastes and which can be used even when one wants to obtain a gastro-resistant pharmaceutical form or a form with modified release. The features of the pharmaceutical form can be modulated by modifying the amount of starch used in the coating.

Tests performed by using the above-mentioned starch showed the gastro-resistant action, the action of protection against humidity and the capability of releasing the active principle in intestinal environment after few minutes.

As shown, additional functional excipients present in the nutraceutical or pharmaceutical composition of the invention are phosphatidylcholine and phosphatidylserine. These compounds belong to the wide class of phospholipids. The phospholipids are molecules structurally very similar to the triglycerides. They consist of a molecule of esterified glycerol in position 1 and 2 with fatty acids. The fatty acids entering the composition of the natural phospholipids can have a length ranging from 12 to 22 carbon atoms; in position 1 generally there is a saturated fatty acid, in position 2 there are unsaturated fatty acids; in position 3 there is a phosphate group which, in turn, is esterified with a complex molecule of various nature such as colin, serin, ethanolamine or inositol. Such molecules are those conferring the name to the phospholipid (phosphatidylcholine, phosphatidylserine, phosphatidylethanolamine and phosphatidylinositol) and influence the physical properties thereof since they establish if the molecule is anionic (such as phosphatidylserine) or zwitterionic (such as phosphatidylcholine). The peculiar chemical structure makes the phospholipids amphiphile molecules, that is molecules capable of interacting both with polar solvents and with apolar solvents. More precisely, the carbonaceous chains of the fatty acids represent the apolar portion interacting with the apolar solvents, whereas the phospholipid head, constituted by the phosphate group and by the molecule bound thereto, represents the polar portion interacting with water and the other polar solvents. This structural feature makes them molecules with surfactant properties which above a certain temperature, defined micellar critical concentration, aggregate by forming characteristic complexes which can vary by shape and size based upon the conditions of the environment in which they form and upon the length of the chains of the fatty acids composing them. For example, if dispersed in aqueous solution, they form micelles with typical shape, with the polar heads facing outwards towards the aqueous environment and the hydrophobic tails facing inwards. If dispersed in organic solvents, they form instead the so-called inverse micelles, wherein the heads are facing inwards and the tails towards the external apolar environment. As far as the sizes are concerned, they vary based upon the length of the carbonaceous chains of the fatty acids composing the phospholipids.

The phospholipids are molecules with a very important biological meaning, first of all since they take part in the composition of the biological membranes and, secondly, since they are involved in different complex mechanisms such as the transduction of the intracellular signals, the adjustment of the intracellular concentration of some ions and the mediation of the inflammatory processes as source of arachidonic acid. The phospholipids are interesting molecules even from the technological point of view and, therefore, they are used in pharmaceutical and nutraceutical field as technological adjuvants in the formulation of carrying systems for several active principles. Many active principles, in fact, can have poor bioavailability due to the difficulty in crossing the barriers and the biological membranes of many body districts. The phospholipids, and in particular the phosphatidylcholine and phosphatidylserine, represent a valid help in the field of the technological strategies for releasing various active principles. One of the advantages of the carrying systems based on phospholipids consists in the compatibility of the phospholipids with the cell membranes, both at mucosal level and at skin level. Phosphatidylcholine and phosphatidylserine, then, act as *enhancers* of intestinal and topic absorption and such action can be ascribed to the following action mechanisms:
- thanks to their properties and their structure, they can merge with the lipids of the stratum corneum and of the membranes and they can perturbate the structure thereof by allowing the passage of various substances;
- in contact with the intestinal fluids they form micelles which contribute in increasing the absorption of the active principles of interest since they extract the lipids from the membranes and alter the rheological properties, fluidity and composition of the membranes by increasing permeability;
- the above-mentioned micelles protect the active principles from chemical and enzymatic degradation and they can be absorbed in the enterohepatic circulation of the biliary salts, together with the mixed micelles of the diet, and be transported in the blood circulation in which they release the incorporated active principles.

The present invention then allows to obtain:
- Increased permeability through the biological membranes
- Controlled release of monacolin K
- Increase in bioavailability of monacolin K

The present invention constitutes a ready and valid intervention useful for the prevention and/or in the treatment of hypercholesterolemia. Such effect is ascribed to the combined action of the substances constituting it. The starch used in the present invention guarantees the controlled release of monacolin K from red yeast rice, the phosphatidylcholine and phosphatidylserine increase the bioavailability thereof thanks to the multi-mechanism action at level of the intestinal mucosa.

The effectiveness of the nutraceutical or pharmaceutical composition, the present invention relates to, is evaluated by means of the experimental protocol described hereinafter.

In order to evaluate the composition capability according claim 1 of releasing in controlled way over time the active principle, monacolin K from red yeast rice, the tests known to the person skilled in the art, such as dissolution and/or disaggregation studies, can be performed by using simulated biological fluids.

In order to evaluate intestinal permeability, an in-vitro test for example on Ca-co-2 cells is performed. The, cultured, cells are prepared by using for example a suitable growth medium (for example containing FBS, fetal bovine serum) and kept under controlled conditions (for example of 37°C, in an atmosphere of 5% CO₂ and 100% humidity). After several passages the cells are washed and pre-incubated with PBS. After the treatment with the formulations of interest the collected samples are subjected to analysis to quantify monacolin K.

The effectiveness of the present invention can be evaluated by monitoring the cholesterol-lowering activity of monacolin K from red yeast rice formulated according to claim 1 of the present invention with respect to monacolin K as such by using methods known to the person skilled in the art such as quantification of total cholesterol, LDL cholesterol, HDL cholesterol, triglycerides in in-vitro or in-vivo models.

The nutraceutical or pharmaceutical composition of the present invention is particularly effective in the prevention and/or the treatment of hypercholesterolemia thanks to the synergic action of the components thereof.

As previously shown, the nutraceutical or pharmaceutical composition of the present invention is inserted within a pharmaceutical product or a food supplement, which is formulated in a suitable dosage form, the composition and preparation thereof is within the capability of the person skilled in the art.

In a preferred embodiment, the red yeast rice containing monacolin K in the nutraceutical or pharmaceutical composition of the invention is present in an amount ranging from 0.1 to 99%, preferably ranging from 0.5 to 98%, still more preferably ranging from 1 to 95% with respect to the total weight of the composition the present invention relates to.

By way of example, additional percentages of red yeast rice containing monacolin K usable in the invention composition are: 2%, 3%, 4%, 6%, 7%, 8%, 9%, 10%, 15%, 20%, 25%, 30%, 35%, 40%, 45%, 50%, 55%, 60%, 65%, 75% or 85%.

In another preferred embodiment, the starch in the nutraceutical or pharmaceutical composition of the invention is present in an amount preferably ranging from 0.1 to 90%, more preferably ranging from 0.5 to 80%, still more preferably ranging from 1 to 70% with respect to the total weight of the composition the present invention relates to.

By way of example, additional percentages of starch usable in the invention composition are: 2%, 3%, 4%, 6%, 7%, 8%, 9%, 10%, 15%, 20%, 25%, 30%, 35%, 40%, 45%, 50%, 55%, 60%, 65%, 75% or 85%.

In still another preferred embodiment, the phosphatidylcholine in the nutraceutical or pharmaceutical composition of the invention is present in an amount ranging from 0.01 to 50%, preferably ranging from 0.05 to 30%, still more preferably ranging from 0.1 to 10% with respect to the total weight of the composition the present invention relates to.

By way of example, additional percentages of phosphatidylcholine usable in the invention composition are: 0.02%, 0.03%, 0.04%, 0.06%, 0.07%, 0.08%, 0.09%, 0.2%, 0,3%, 0.4%, 0.6%, 0.7%, 0.8%, 0.9%, 1%, 2%, 3%, 4%, 5%, 6%, 7%, 8%, 9%, 15%, 20%, 25%, 35%, 40% or 45%.

In still another preferred embodiment, the phosphatidylserine in the nutraceutical or pharmaceutical composition of the invention is present in an amount ranging from 0.01 to 50%, preferably ranging from 0.05 to 30%, still more preferably ranging from 0.1 to 10% with respect to the total weight of the composition the present invention relates to.

By way of example, additional percentages of phosphatidylserine usable in the invention composition are: 0.02%, 0.03%, 0.04%, 0.06%, 0.07%, 0.08%, 0.09%, 0.2%, 0,3%, 0.4%, 0.6%, 0.7%, 0.8%, 0.9%, 1%, 2%, 3%, 4%, 5%, 6%, 7%, 8%, 9%, 15%, 20%, 25%, 35%, 40% or 45%.

All above-mentioned preferred embodiments can be combined with each other.

The pharmaceutical product or food supplement, which comprises the pharmaceutical or nutraceutical composition of the invention, is formulated in a preferably oral pharmaceutical form, which can be solid, semi-solid or liquid.

By way of example a powder, an orosoluble powder, a granulate, a hard capsule, a soft-gel capsule, a tablet, a sachet, a solution, a syrup, a suspension or an emulsion can be mentioned.

Some not limiting examples of nutraceutical or pharmaceutical compositions, the present invention relates to, are provided hereinafter. As mentioned above, such nutraceutical or pharmaceutical compositions are formulated as pharmaceutical products or food supplements and administered in a suitable oral dosage form, in case divided into one or more dosage units such as, for example, a capsule, a tablet or a sachet.

The following examples are provided by pure way of not limiting example of the invention scope as defined by the enclosed claims.

### EXAMPLES

### EXAMPLE 1

| ***Active principle*** | ***Amount %*** |
|---|---|
| Red yeast rice containing monacolin K | 92.2 |
| Acetylated MODIFIED STARCH | 6 |
| Phosphatidylcholine 30% | 1.2 |
| Phosphatidylserine 60% | 0.6 |

### EXAMPLE 2

| ***Active principle*** | ***Amount %*** |
|---|---|
| Red yeast rice containing monacolin K | 54 |
| Acetylated MODIFIED STARCH | 44.2 |
| Phosphatidylcholine 30% | 1.2 |
| Phosphatidylserine 60% | 0.6 |

### EXAMPLE 3

| ***Active principle*** | ***Amount %*** |
|---|---|
| Red yeast rice containing monacolin K | 80 |
| Acetylated MODIFIED STARCH | 12 |
| Phosphatidylcholine 30% | 2.4 |
| Phosphatidylserine 60% | 1.2 |

### EXAMPLE 4

| ***Active principle*** | ***Amount %*** |
|---|---|
| Red yeast rice containing monacolin K | 40 |
| PREGELATINIZED MAIZE STARCH | 55 |
| Phosphatidylcholine 30% | 3.6 |
| Phosphatidylserine 60% | 1.4 |

### EXAMPLE 5

| ***Active principle*** | ***Amount %*** |
|---|---|
| Red yeast rice containing monacolin K | 60 |
| Acetylated MODIFIED STARCH | 35 |
| Phosphatidylcholine 30% | 2.5 |
| Phosphatidylserine 60% | 2.5 |

### EXAMPLE 6

For the preparation of the product "Red rice containing granular monacolin K (according to claim 1)" it is possible to use for example the fluid-bed granulation technology. A preparation example, applied to the compositions of the Examples, is provided hereinafter.

The working process consists in the following steps:
a) Mixing:
   The raw materials previously loaded in the granulator basket are subjected to a first mixing step on fluid bed, with process air having a determined temperature (for example 80-90°C), until obtaining a mixture having an average temperature of about 44°C. During this step one proceeds with implementing a homogeneous bulk from the point of view of composition and temperature, prerequisite necessary for the optimal course of the subsequent granulation step.
b) Granulation
   The granulation step provides the insertion of an aqueous solution of a suitably selected binding or granulating agent, by direct nebulization on the premixed bulk, fluidized on fluid bed. Even in this step process air for example at 90°C is used by selecting suitably the speed for entering the binding solution to obtain a granular structured according to expectations (granulometry, bulk density, smoothness) and homogeneous.
c) Drying
   During the drying step the water content of the preformed granular is brought back tendentially to the conditions of the mixture of the starting raw materials. The temperature of the process air of the granulate at the end of the step is suitably evaluated during pilot tests depending on such objective.
d) Calibration
   The half-finished product obtained from the preceding step is transferred from the fluid bed granulator to a swinging granulator where it is calibrated through a sieve for the reduction in the particle size of the granules and of the agglomerates with coarser structure.

## Claims

1. A nutraceutical or pharmaceutical composition comprising the combination of an extract of red yeast rice containing monacolin K, phosphatidylcholine, phosphatidylserine and starch.

2. The nutraceutical or pharmaceutical composition according to claim 1, for use in the treatment or prevention of hypercholesterolemia in both humans and animals.

3. The nutraceutical or pharmaceutical composition for use according to claims 1 and 2, comprising an amount of an extract of red yeast rice containing monacolin K ranging from 0.1 to 99%, preferably ranging from 0.5 to 98%, still more preferably ranging from 1 to 95% with respect to the total weight of the above-mentioned combination.

4. The nutraceutical or pharmaceutical composition for use according to any one of claims 1 to 3, comprising starch, preferably acetylated pregelatinized starch, preferably in an amount ranging from 0.1 to 90%, more preferably ranging from 0.5 to 80%, still more preferably ranging from 1 to 70% with respect to the total weight of the above-mentioned combination.

5. The nutraceutical or pharmaceutical composition for use according to any one of claims 1 to 4, comprising an amount of phosphatidylcholine ranging from 0.01 to 50%, preferably ranging from 0.05 to 30%, still more preferably ranging from 0.1 to 10% with respect to the total weight of the above-mentioned combination and an amount of phosphatidylserine ranging from 0.01 to 50%, preferably ranging from 0.05 to 30%, still more preferably ranging from 0.1 to 10% with respect to the total weight of the above-mentioned combination.

6. The pharmaceutical product or food supplement according to claims 1 to 5, formulated in a liquid, semi-solid or solid oral dosage form.

7. The pharmaceutical product or food supplement according to claim 6, in which the dosage form is a powder, an orosoluble powder, a granulate, a hard capsule, a soft-gel capsule, a tablet, a sachet, a solution, a syrup, a suspension or an emulsion.

## Patentansprüche

1. Nutrazeutische oder pharmazeutische Zusammensetzung, umfassend die Kombination aus einem Extrakt aus rotem Hefereis, der Monacolin K enthält, Phosphatidylcholin, Phosphatidylserin und Stärke.

2. Nutrazeutische oder pharmazeutische Zusammensetzung nach Anspruch 1 zur Verwendung bei der Behandlung oder der Prävention von Hypercholesterinämie sowohl bei Menschen als auch bei Tieren.

3. Nutrazeutische oder pharmazeutische Zusammensetzung zur Verwendung nach den Ansprüchen 1 und 2, umfassend eine Menge eines Extrakts aus rotem Hefereis, der Monacolin K enthält, im Bereich von 0,1 bis 99 %, vorzugsweise im Bereich von 0,5 bis 98 %, noch mehr bevorzugt im Bereich von 1 bis 95 %, bezogen auf das Gesamtgewicht der oben genannten Kombination.

4. Nutrazeutische oder pharmazeutische Zusammensetzung zur Verwendung nach einem der Ansprüche 1 bis 3, umfassend Stärke, vorzugsweise acetylierte prägelatinierte Stärke, vorzugsweise in einer Menge im Bereich von 0,1 bis 90 %, mehr bevorzugt im Bereich von 0,5 bis 80 %, noch mehr bevorzugt im Bereich von 1 bis 70 %, bezogen auf das Gesamtgewicht der oben genannten Kombination.

5. Nutrazeutische oder pharmazeutische Zusammensetzung zur Verwendung nach einem der Ansprüche 1 bis 4, umfassend eine Menge an Phosphatidylcholin im Bereich von 0,01 bis 50 %, vorzugsweise im Bereich von 0,05 bis 30 %, noch mehr bevorzugt im Bereich von 0,1 bis 10 %, bezogen auf das Gesamtgewicht der oben genannten Kombination, und eine Menge an Phosphatidylserin im Bereich von 0,01 bis 50 %, vorzugsweise im Bereich von 0,05 bis 30 %, noch mehr bevorzugt im Bereich von 0,1 bis 10 %, bezogen auf das Gesamtgewicht der oben genannten Kombination.

6. Pharmazeutisches Produkt oder Nahrungsergänzungsmittel nach den Ansprüchen 1 bis 5, formuliert in einer flüssigen, halbfesten oder festen oralen Dosierungsform.

7. Pharmazeutisches Produkt oder Nahrungsergänzungsmittel nach Anspruch 6, wobei die Dosierungsform ein Pulver, ein im Mund lösliches Pulver, ein Granulat, eine Hartkapsel, eine Weichgelkapsel, eine Tablette, einen Beutel, eine Lösung, ein Sirup, eine Suspension oder eine Emulsion ist.

## Revendications

1. Composition nutraceutique ou pharmaceutique comprenant la combinaison d'un extrait de levure de riz rouge contenant de la monacoline K, de la phosphatidylcholine, de la phosphatidylsérine et de l'amidon.

2. Composition nutraceutique ou pharmaceutique selon la revendication 1, pour utilisation dans le traitement ou la prévention d'hypercholestérolémie à la fois chez les humains et chez les animaux.

3. Composition nutraceutique ou pharmaceutique pour utilisation selon les revendications 1 et 2, comprenant une quantité d'un extrait de levure de riz rouge contenant de la monacoline K allant 0,1 à 99 %, de préférence allant de 0,5 à 98 %, encore plus préférablement allant de 1 à 95 % par rapport au poids total de la combinaison précitée.

4. Composition nutraceutique ou pharmaceutique pour utilisation selon l'une quelconque des revendications 1 à 3, comprenant de l'amidon, de préférence de l'amidon prégélatinisé acétylé, de préférence en une quantité allant de 0,1 à 90 %, plus préférablement allant de 0,5 à 80 %, encore plus préférablement allant de 1 à 70 % par rapport au poids total de la combinaison précitée.

5. Composition nutraceutique ou pharmaceutique pour utilisation selon l'une quelconque des revendications 1 à 4, comprenant une quantité de phosphatidylcholine allant de 0,01 à 50 %, de préférence allant de 0,05 à 30 %, encore plus préférablement allant de 0,1 à 10 % par rapport au poids total de la combinaison précitée et une quantité de phosphatidylsérine allant de 0,01 à 50 %, de préférence allant de 0,05 à 30 %, encore plus préférablement allant de 0,1 à 10 % par rapport au poids total de la combinaison précitée.

6. Produit pharmaceutique ou complément alimentaire selon les revendications 1 à 5, formulé sous une forme galénique orale liquide, semi-solide ou solide.

7. Produit pharmaceutique ou complément alimentaire selon la revendication 6, dans lequel la forme galénique est une poudre, une poudre soluble en bouche, un granulé, une gélule dure, une gélule molle, un comprimé, un sachet, une solution, un sirop, une suspension ou une émulsion.
